# EUROPEAN PATENT APPLICATION

(11) **EP 4 751 746 A1**
(43) Date of publication of application: **03.06.2026**
(21) Application number: 24845664.2
(22) Date of filing: 25.07.2024
(51) Int. Cl.: A61M 1/36, A61P 25/16, B01D 15/00, B01J 20/22, C08B 37/12

(54) **POROUS MOLDED BODY FOR ALPHA-SYNUCLEIN OLIGOMER REMOVAL, ALPHA-SYNUCLEIN OLIGOMER REMOVAL METHOD, AND ALPHA-SYNUCLEIN OLIGOMER REMOVAL SYSTEM**

(30) Priority: 27.07.2023 JP 2023122589
(71) Applicant: Asahi Kasei Medical Co., Ltd., Tokyo 100-0006 (JP)
(72) Inventor: TAKATSUJI, Ryo, Tokyo 100-0006 (JP); KAYUKAWA, Taiki, Tokyo 100-0006 (JP)
(74) Representative: dompatent
(86) International application number: PCT/JP2024/026561
(87) International publication number: WO 2025/023289

(57) **Abstract**

The present disclosure provides a porous molded body, method, and system enabling removal of α-synuclein oligomers from a biological fluid. The porous molded body for α-synuclein oligomer removal according to the present disclosure includes a porous molded body carrier having a positive charge due to having an amine structure. The α-synuclein oligomer removal method according to the present disclosure includes bringing the porous molded body according to the present disclosure into contact with a biological fluid containing α-synuclein oligomers. The α-synuclein oligomer removal system according to the present disclosure comprises: a column which has a container that has an inlet and outlet for biological fluid, and a porous molded body that is loaded into the container; a biological fluid introduction flow path into which the biological fluid is introduced through the inlet; and a biological fluid discharge flow path from which the biological fluid is discharged through the outlet. The porous molded body is a porous molded body carrier having a positive charge due to having an amine structure.

## Description

### TECHNICAL FIELD

The present disclosure relates to a porous molded body for α-synuclein oligomer removal, to an α-synuclein oligomer removal method, and to an α-synuclein oligomer removal system.

### BACKGROUND ART

Parkinson's disease is a progressive disorder of the central nervous system characterized by decreased spontaneous movement, walking difficulty, unstable posture, muscular stiffness and tremors (shaking). Parkinson's disease is caused by degeneration of dopamine-containing pigmented neurons in the substantia nigra of the midbrain, and it is known that this degeneration lowers the dopamine concentration in the striatum. In pathological terms, Parkinson's disease is characterized by the presence of Lewy bodies primarily in the substantia nigra, the major components of Lewy bodies being filaments composed of α-synuclein. Since early-onset familial Parkinson's disease patients have the major mutations of two α-synuclein amino acids, these Lewy bodies are believed to contribute to neurodegeneration in Parkinson's disease and related diseases.

Multiple system atrophy is a progressive neurodegenerative disease characterized by degeneration of neurons in the cerebellar cortex, pontine nuclei, olivary nuclei, striatum, substantia nigra, autonomic nuclei of the brainstem or spinal cord, as well as the motor area of the cerebral cortex, and also by accumulation of inclusion bodies composed of insolubilized α-synuclein in the oligodendroglial cytoplasm. Compared to Parkinson's disease, the symptom of tremor at rest is milder, progression is more rapid and anti-Parkinson's drugs are less effective.

Lewy body dementia is a disease characterized by appearance of Lewy bodies in the brain, with the major symptoms being cognitive impairment, hallucination and delusion, and sleep disorder. Although motor function impairment characteristic of Parkinson's disease is sometimes observed, it differs from Parkinson's disease in that cognitive function is usually reduced as an initial symptom. In Lewy body dementia, the Lewy bodies are usually distributed across a wide region including the cerebral cortex, thalamus and brainstem of the brain.

The current standard for treatment of motor function impairment in synucleinopathies such as Parkinson's disease, multiple system atrophy and Lewy body dementia is administration of L-dopa, which is a dopamine precursor molecule. L-dopa passes through the blood-brain barrier and is converted to dopamine in the brain. This agent alleviates symptoms associated with dopamine deficiency, but cannot stop the progressive neurodegeneration which is typical of synucleinopathy. In order to block progression of synucleinopathy it is necessary to elucidate the mechanism by which shedding of neurons occurs, but at the current time no fundamental treatment method or therapeutic agent exists. In Japan, synucleinopathy has been indicated as an intractable disease (specific disease), being a disease with few cases but long-term impairment in terms of quality of life.

Although much still remains unknown regarding the physiological function of α-synuclein as a component of Lewy bodies, it has been reported to play a protective role against viral infection, in addition to being involved in membrane fusion and maintenance and release of synaptic vesicles. When α-synuclein monomers aggregate under some conditions to form α-synuclein oligomers, this creates a pathogenic condition which leads to impaired calcium regulation, mitochondrial dysfunction and intracellular transport disorder.

The process of aggregation of α-synuclein monomers in the body to form α-synuclein oligomers is not fully understood at the time of the present filing, but in recent years the "α-synuclein oligomer propagation" hypothesis has been put forth, suggesting that α-synuclein oligomers may propagate between cells based on properties of prion proteins, thus creating the condition of "synucleinopathy". An "auto-templating phenomenon" is observed *in vitro,* whereby α-synuclein monomers having normal structure progressively aggregate using α-synuclein oligomers with a β-sheet structure as the template. It has also been reported that in animal experiments using mice, administration of α-synuclein oligomers through the brain, or even intraperitoneal, oral or intravenous administration, results in neurologic symptoms, with aggregates of α-synuclein being seen in the central nervous system (NPL 1).

Although the "α-synuclein oligomer propagation" hypothesis does not completely explain the pathway through which α-synuclein oligomers would propagate, it has been reported that large amounts of α-synuclein oligomers are present in the blood plasma of Parkinson's disease patients compared to healthy persons (NPL 2).

PTL 1 describes means for removing free α-synuclein monomers in body fluids before they accumulate in neurons, by adsorption removal. The means described in PTL 1 is for the purpose of adsorption removal of only α-synuclein monomers using a ligand-introduced porous support, while it does not describe means that can potentially delay progression of Parkinson's disease, multiple system atrophy and/or Lewy body dementia by removal of α-synuclein oligomers from biological fluids.

### [CITATION LIST]

### [PATENT LITERATURE]

[PTL 1] Japanese Unexamined Patent Publication No. 2012-193112

### [NON PATENT LITERATURE]

[NPL 1] LOHMANN, Stephanie, et al., "Oral and intravenous transmission of α-synuclein fibrils to mice", Acta Neuropathologica, (2019), 138: 515-533.
[NPL 2] EL-AGNAF, Omar MA, et al., "Detection of oligomeric forms of α-synuclein protein in human plasma as a potential biomarker for Parkinson's disease", The FASEB journal, (2006), 20(3): 419-425.

### SUMMARY

### [TECHNICAL PROBLEM]

It is an object of the present disclosure to provide a porous molded body, method and system that allow removal of α-synuclein oligomers from biological fluids.

### [SOLUTION TO PROBLEM]

The following are examples of embodiments of the disclosure.
[1] A porous molded body for removal of α-synuclein oligomers, comprising a porous molded support having a positive charge derived from an amine structure, wherein the porous molded support comprises the amine structure at 30 µmol/ml-wet support or greater and 10,000 µmol/ml-wet support or less.
[2] The porous molded body according to [1], wherein the amine structure is an aliphatic amine structure.
[3] The porous molded body according to [1] or [2], wherein the amine structure is a secondary, tertiary or quaternary amine, or a combination thereof, and the amine group has 1 to 6 carbon atoms.
[4] The porous molded body according to any one of [1] to [3], wherein the amine structure includes a quaternary amine.
[5] The porous molded body according to any one of [1] to [4], wherein the porous molded support is made of crosslinked agarose, crosslinked cellulose or a polystyrene-based polymer, or a combination thereof.
[6] The porous molded body according to any one of [1] to [5], wherein the harmonic mean particle size of the porous molded support is 100 µm to 800 µm.
[7] The porous molded body according to any one of [1] to [6], wherein the specific surface area of the porous molded support is 1 m²/g to 100 m²/g.
[8] An α-synuclein oligomer removal system, the system comprising:
   a column having a container with an inlet and outlet for a biological fluid, and a porous molded body filled into the container,
   a biological fluid introduction channel through which the biological fluid is introduced into the inlet, and
   a biological fluid discharge channel through which the biological fluid that has passed through the column is discharged from the outlet,
   wherein the porous molded body comprises a porous molded support having a positive charge derived from an amine structure, the porous molded support having the amine structure at 30 µmol/ml-wet support or greater and 10,000 µmol/ml-wet support or less.
[9] The system according to [8], wherein the biological fluid is blood, and a plasma separator is provided between the column and the biological fluid introduction channel, the construction being such that plasma separated from the blood flows through the column.
[10] A method for removing α-synuclein oligomers from a biological fluid, wherein the method comprises:
   contacting a porous molded body according to any one of [1] to [7] with a biological fluid containing the α-synuclein oligomers.
[11] The method according to [10], wherein the biological fluid is blood or plasma.
[12] A method for lowering the α-synuclein oligomer concentration in a biological fluid of a patient, wherein the method comprises:
   harvesting a biological fluid from the patient's body,
   contacting the harvested biological fluid with a porous molded body and obtaining the contacted biological fluid, and
   returning the contacted biological fluid back to the patient's body,
   the porous molded body comprising a porous molded support having a positive charge derived from an amine structure, and the porous molded support having the amine structure at 10 µmol/ml-wet support or greater and 3000 µmol/ml-wet support or less.
[13] The method according to [12], wherein the biological fluid is blood or plasma.

### [ADVANTAGEOUS EFFECTS OF INVENTION]

According to the present disclosure it is possible to provide a porous molded body, method and system that allow removal of α-synuclein oligomers from biological fluids.

### DESCRIPTION OF EMBODIMENTS

### <Porous molded body>

The porous molded body of the disclosure is a porous molded body for removal of α-synuclein oligomers. The porous molded body comprises a porous molded support having a positive charge (plus charge) due to its amine structure, allowing α-synuclein oligomers to be removed from a biological fluid. The present inventors have conducted much diligent research on means for removing α-synuclein oligomers based on the α-synuclein oligomer propagation hypothesis (see NPLs 1 and 2), believing that removal of α-synuclein oligomers from biological fluids to block the pathway of propagation of α-synuclein oligomers may be effective for inhibiting progression of synucleinopathy. As a result, the present inventors have found, as a previously unknown attribute, that a porous molded support having a positive charge derived from an amine structure can adsorb and remove α-synuclein oligomers, and the present invention has been completed based on this finding.

### [Porous molded support]

The porous molded support is positively charged due to an amine structure which is bonded to the porous molded support. The amount of the amine structure on the porous molded support is preferably 10 µmol/ml-wet support or greater and 10,000 µmol/ml-wet support or less, more preferably 20 µmol/ml-wet support or greater and 10,000 µmol/ml-wet support or less, even more preferably 30 µmol/ml-wet support or greater and 10,000 µmol/ml-wet support or less, yet more preferably 30 µmol/ml-wet support or greater and 5000 µmol/ml-wet support or less, even yet more preferably 40 µmol/ml-wet support or greater and 3000 µmol/ml-wet support or less, and most preferably 50 µmol/ml-wet support or greater and 2000 µmol/ml-wet support or less. If the amine structure content is 10 µmol/ml-wet support or greater, then the effect of removing the α-synuclein oligomers will be further augmented. While a greater amount of introduced amine structure is preferred from the viewpoint of adsorption of α-synuclein oligomers, the amount of the amine structure is preferably 10,000 µmol/ml-wet support or less and more preferably 400 µmol/ml-wet support or less from the viewpoint of facilitating introduction of the amine structure and reducing the necessary amount of reagent for the introduction reaction in order to lower cost.

A porous molded support is a support having a porous structure with numerous pores of suitable sizes. The sizes of the pores of the porous molded support are not particularly restricted, but the specific surface area is preferably 0.0001 m²/g to 1000 m²/g, more preferably 1 m²/g to 100 m²/g and even more preferably 1 m²/g to 50 m²/g. If the specific surface area is 1000 m²/g or lower the mechanical strength of the porous molded support will be increased, while if it is 0.0001 m²/g or higher the adsorption efficiency will tend to be increased, and therefore this range results in a more satisfactory balance between adsorption efficiency and mechanical stability. The shape of the porous molded support is not particularly restricted, and may be in any desired form such as particulate (beads), filamentous, sheet-like, hollow fiber, cylindrical or hollow cylindrical, for example, or a monolith support in which at least some of the open holes penetrate through the support. The porous molded support is preferably particulate (beads). For a porous molded support which is particulate (beads), the particle sizes are not particularly restricted but the harmonic mean particle size is preferably 20 µm to 1500 µm, more preferably 50 µm to 1000 µm and even more preferably 100 µm to 800 µm. When used in an α-synuclein oligomer removal system as described below, a harmonic mean particle size of 20 µm or larger will improve the flow property of the biological fluid, while a value of 1500 µm or smaller will tend to increase the specific surface area per volume and improve the adsorption efficiency, thus resulting in a more satisfactory balance between the flow property and adsorption efficiency.

The material composing the porous molded support is not particularly restricted so long as it is a porous body allowing introduction of an amine structure, and it may be an inorganic compound or organic compound, for example. The material of the porous molded support is preferably an organic polymer, since this will help reduce elution in hot water and will allow easier and more precise control of the pores in the porous body. Examples of such organic polymers include crosslinked or non-crosslinked synthetic polymer supports such as polyvinyl alcohol, polyacrylate, polymethacrylate, polyacrylamide, polyacrylic acid, polyamide, polyester, polyethylene and polystyrene; copolymer supports comprising two or more of these; crosslinked or non-crosslinked polysaccharide supports such as cellulose, agarose (Sepharose) and dextrin; and composite supports such as organic-organic and organic-inorganic substances obtained by combinations of the foregoing. Cellulose may also be crystalline cellulose. Preferred among these materials are materials with satisfactory blood compatibility, relatively low nonspecific adsorption and satisfactory adsorption selectivity for α-synuclein oligomers. Examples of such preferred materials include crosslinked agarose, crosslinked cellulose and polystyrene-based polymers, as well as their combinations.

### [Amine structure]

For the purpose of the disclosure, "amine structure" refers to a structure having one or more amino groups on a hydrocarbon group. The "amine structure" is preferably an "aliphatic amine structure". An "aliphatic amine structure" is a structure having one or more amino groups on the aliphatic group of a hydrocarbon group comprising or entirely composed of an aliphatic group. The hydrocarbon group may have an aromatic group in addition to the aliphatic group, with the amino group being directly bonded to the aliphatic group or directly bonded to the aromatic group. In the case of an aliphatic amine structure, the amino group is directly bonded on the aliphatic group. An amino group may be an unsubstituted amino group (primary amine) or a substituted amino group (secondary, tertiary or quaternary amine). If at least some or all of the amino groups are in the form of ammonium ions, i.e. primary, secondary, tertiary or quaternary ammonium groups, the structure will be positively charged at the pH of biological fluids. The porous molded support may have an amine structure either via covalent bonding or via non-covalent bonding.

When the porous molded support has an amine structure via covalent bonding, the amino group in the form of ammonium ion can be represented by the following general formula (1):
[Chemical Formula 1]

-N⁺R¹R²R³ ... (1)

for example. In general formula (1), "bonding" (-) means bonding to a hydrocarbon group (also referred to herein as "linker structure"). Preferably, R¹, R² and R³ each independently represent a hydrogen atom or a hydrocarbon group. Preferably, one or more, two or more, three or more or all four of R¹ , R² and R³ are hydrocarbon groups. The hydrocarbon groups R¹, R² and R³ may be saturated or unsaturated, and may have straight-chain, branched-chain or cyclic structures, and may be aliphatic, aromatic, or a combination thereof. The number of carbon atoms of the hydrocarbon groups of R¹, R² and R³ is not restricted, but is preferably 1 to 20, more preferably 1 to 15, even more preferably 1 to 10, yet more preferably 1 to 8 and most preferably 1 to 6. The hydrocarbon groups R¹, R² and R³ may have heteroatom groups, and for example, they may have hydroxyl, halogen, amino, carbonyl or sulfonyl groups or ether bonds in part of the structure.

Examples of amine compounds that provide an amine structure as in general formula (1) include dimethylamine, trimethylamine, ethylamine, diethylamine, triethylamine, butylamine, N,N-dimethylbutylamine, N,N-dimethylhexylamine, N-benzyl-N-methyl-ethanolamine, N,N-dimethyl-2-phenoxyethylamine, hexadecylamine (cetylamine) and polyethyleneimine.

When the porous molded support has an amine structure via covalent bonding, and the amine structure has multiple amino groups, the amine structure can be represented by the following general formula (2):
[Chemical Formula 2]

-N(R⁴)-[R⁵-N(R⁶)]ₙ-R⁷ ... (2)

for example. At least one amino group in general formula (2) has the form of an ammonium ion at the pH of biological fluids, and it may have a positive charge. The leftmost bond "-" is the bond of the linker structure to a hydrocarbon group. Here, "n" is an integer of 1 to 100, preferably 1 to 50, more preferably 1 to 30, even more preferably 1 to 10 and most preferably 1 to 5. R⁴, R⁶ and R⁷ each independently represent a hydrogen atom or a monovalent hydrocarbon group. The hydrocarbon groups R⁴, R⁶ and R⁷ may be saturated or unsaturated, and may have straight-chain, branched-chain or cyclic structures, and may be aliphatic, aromatic, or a combination thereof. The number of carbon atoms of the hydrocarbon groups of R⁴, R⁶ and R⁷ is not restricted, but is preferably 1 to 20, more preferably 1 to 15, even more preferably 1 to 10, yet more preferably 1 to 8 and most preferably 1 to 6. The hydrocarbon groups R⁴, R⁶ and R⁷ may have heteroatom groups, and for example, they may have hydroxyl, halogen, amino, carbonyl or sulfonyl groups or ether bonds in part of the structure. At least one of R⁴, R⁶ and R⁷ is preferably a hydrogen atom, and more preferably all are hydrogen atoms (-NH(R⁵NH)ₙH). Each R⁵ independently represents a divalent hydrocarbon group. The R⁵ hydrocarbon groups may be saturated or unsaturated, and may have straight-chain, branched-chain or cyclic structures, and may be aliphatic, aromatic or combinations thereof. The number of carbon atoms of the R⁵ hydrocarbon groups is not restricted, but is preferably 1 to 20, more preferably 1 to 15, even more preferably 1 to 10, yet more preferably 1 to 8 and most preferably 1 to 6. The R⁵ hydrocarbon groups may have heteroatom groups, and for example, they may have hydroxyl, halogen, amino, carbonyl or sulfonyl groups or ether bonds in part of the structure. R⁵ is preferably a divalent alkylene group, examples of which include substituted or unsubstituted methylene, ethylene, propylene and butylene groups. Preferred examples for the structure of general formula (2) include -NH(CH₂NH)ₙH, -NH(CH₂CH₂NH)ₙH and - NH(CH2CH2CH2NH)ₙH.

A smaller number of carbon atoms for a hydrocarbon group bonded to an amino group (R¹, R² or R³ in general formula (1)) in the amine structure will tend to exhibit electrostatic interaction between the positive charge of the amine structure and the α-synuclein oligomers, thus allowing the α-synuclein oligomers to be more effectively removed. From an economical standpoint as well, a smaller number of carbon atoms in the hydrocarbon group bonded to the amino group will tend to reduce the cost of reagent per amino group for introduction of the amine structure. Therefore, when the amine structure is a secondary, tertiary or quaternary amine, or a combination of these, and defining the "number of amine carbons" to be the maximum number of carbon atoms among the hydrocarbon groups (R¹, R² or R³ in general formula (1)) excluding the linker among the hydrocarbon chains bonded to the amino group, then the amine group preferably has 1 to 6 carbon atoms. Examples of amine compounds that provide amine structures with such numbers of amine carbons include dimethylamine, trimethylamine, ethylamine, diethylamine, triethylamine, butylamine, N,N-dimethylbutylamine and N,N-dimethylhexylamine.

The amine structure preferably includes a quaternary amine regardless of the number of amine carbons. This will increase the proportion of amine structures that are positively charged at the pH of biological fluids, thus helping to exhibit electrostatic interaction between the positive charge of the amine structure and the α-synuclein oligomers, and allowing the α-synuclein oligomers to be more effectively removed. Examples of amine compounds that provide such quaternary amine structures include trimethylamine, triethylamine, N,N-dimethylbutylamine, N,N-dimethylhexylamine, N-benzyl-N-methyl-ethanolamine and N,N-dimethyl-2-phenoxyethylamine.

The linker structure may be a divalent hydrocarbon group having a saturated or unsaturated straight-chain, branched-chain or cyclic structure. The number of carbon atoms of the hydrocarbon group used as the linker structure is not restricted, but is preferably 1 to 20, more preferably 1 to 15, even more preferably 1 to 10, yet more preferably 1 to 8 and most preferably 1 to 6. The hydrocarbon groups in the linker structures may have heteroatoms, and for example, they may have hydroxyl, halogen, amino, carbonyl or sulfonyl groups or ether bonds in part of the structure. More specific examples of linker structures include substituted or unsubstituted alkylenes such as methylene, ethylene, propylene and butylene.

Examples of compounds that provide a linker structure (linkers) include epichlorhydrin, epibromohydrin, allyl-glycidyl ether, bis-epoxides (such as butanediol diglycidyl ether), halogen-substituted aliphatic substances (such as dichloropropanol and allyl halides), and divinylsulfone. When the porous molded support has a hydroxyl group, for example, an allyl halide such as allyl bromide may be used as the linker.

As an example in which the porous molded support has an amine structure via non-covalent bonding, the porous molded support may have a polymer with an amine structure on its surface. The polymer with an amine structure preferably has the aforementioned amine structure on the main chain of the polymer and/or as a side chain. More specifically, the polymer with an amine structure may be a polyalkyleneimine such as polyethyleneimine.

### [α-Synuclein oligomer]

An "α-synuclein oligomer" may be an oligomer or aggregate formed from two or more α-synuclein monomers, or its phosphorylated form. The porous molded body of the disclosure is a porous molded body for removal of α-synuclein oligomers from biological fluids. The porous molded body of the disclosure is suitable for contact with a biological fluid to remove the α-synuclein oligomers from the biological fluid. Biological fluids generally contain sufficient amounts of low-molecular-weight substances that can be adsorbed onto porous molded bodies, and since low-molecular-weight substances rapidly occupy the accessible adsorption sites of the porous molded body, adsorption removal of high-molecular-weight substances tends to be difficult using porous molded bodies. Moreover, substances to be adsorbed which have significantly high molecular weight do not permeate to the interiors of the pores of the porous molded body but are limited to adsorption sites on the surface of the porous molded body, thus tending to hinder their removal by adsorption. The porous molded body of the disclosure, however, is designed to overcome this problem by having the ability to effectively remove even α-synuclein oligomers of large molecular weight. More specifically, while there is no limitation to the molecular weights of α-synuclein oligomers to be removed by the porous molded body of the disclosure, it is possible to remove α-synuclein oligomers of 500,000 kDa and lower, for example, with particularly efficient removal of α-synuclein oligomers of 100,000 kDa and lower. By allowing efficient removal of α-synuclein oligomers, it is possible to delay progression of synucleinopathies such as Parkinson's disease, multiple system atrophy and/or Lewy body dementia. The porous molded body of the disclosure is therefore preferably used to delay progression of synucleinopathy.

The porous molded body of the disclosure may also be used for a formulation, such as for production of a blood preparation using blood from which α-synuclein oligomers have been removed, for example. Alternatively, the porous molded body of the disclosure may be used for diagnosis, such as for concentration and collection of α-synuclein oligomers in a biological fluid, for their detection in diagnosis of synucleinopathy.

### [Biological fluid]

The biological fluid is preferably blood, plasma, serum, spinal fluid, ascites fluid, urine, saliva, lacrimal fluid, nasal discharge, sweat, digestive juice or breast milk, with blood or plasma being especially preferred.

### <Method for producing porous molded body>

The method for producing the porous molded body of the disclosure is not restricted. The method for producing a porous molded body of the disclosure may be, for example, a method that comprises introducing an amine structure onto the surface of a porous molded support which lacks an amine structure. Alternatively, the method may include synthesizing the porous polymer support by polymerization reaction from a monomer having an amine structure or its precursor structure. The details regarding the porous molded support and amine structure are described above and will not be repeated here.

The method of introducing the amine structure onto the surface of a porous molded support lacking an amine structure may be a fixed introduction method by covalent bonding, or a non-fixed introduction method by non-covalent bonding. One example of a fixed introduction method via covalent bonding is a method in which a linker compound that provides a linker structure is reacted with the porous molded support, and then an amine compound (ligand) that provides an amine structure is reacted to introduce a linker structure between the porous molded support and the amine compound. The amine compounds and linkers were described in detail above and will not be described again here.

One example of a non-fixed introduction method via non-covalent bonding comprises coating the surface of a porous molded support with a polymer having an amine structure (such as polyethyleneimine). The method of coating the polymer with an amine structure onto the surface of the porous molded support may be any desired coating method, such as an application method, spraying method or dipping method. Details regarding the polymer with an amine structure are described above and will not be repeated here.

### <Method for removing α-synuclein oligomers>

The method for removing α-synuclein oligomers according to the disclosure comprises contacting the porous molded body of the disclosure with a biological fluid which contains α-synuclein oligomers. The manner of contact is not particularly restricted so long as the concentration of α-synuclein oligomers in the biological fluid can be reduced. For example, the removal method preferably comprises introducing an α-synuclein oligomer-containing biological fluid through the inlet of a container which has an inlet and outlet for biological fluids and is filled with a porous molded body of the disclosure, contacting the biological fluid with the porous molded body of the disclosure and causing it to pass through the container, and discharging the biological fluid from the porous molded body of the disclosure through the outlet. The removal method may further comprise sampling a biological fluid from the patient's body, and returning the biological fluid that has been contacted with the porous molded body of the disclosure back into the patient's body.

The term "removal" as used herein does not mean complete removal of α-synuclein oligomers from the biological fluid, as it is sufficient if the concentration of the α-synuclein oligomers in the biological fluid can be reduced. The removal method can remove α-synuclein oligomers in a biological fluid by preferably 5% or greater, 10% or greater, 15% or greater, 20% or greater, 25% or greater or 30% or greater.

The biological fluid is most preferably blood or plasma, as mentioned above. In this case, the removal method may comprise contacting blood containing α-synuclein oligomers with the porous molded body of the disclosure to obtain blood from which the α-synuclein oligomers have been removed. Alternatively, the removal method may further comprise separating α-synuclein oligomer-containing plasma from blood beforehand, contacting the separated plasma with the porous molded body of the disclosure to remove the α-synuclein oligomers from the blood plasma, and combining the blood plasma from which the α-synuclein oligomers have been removed with the separated blood cell concentrate, to obtain blood with the α-synuclein oligomers removed. The removal method may further comprise removing blood from the body of a patient and/or returning the blood back into the body of the patient after contacting it with the porous molded body of the disclosure.

### <α-Synuclein oligomer removal system>

The porous molded body of the disclosure can be incorporated into an α-synuclein oligomer removal system. The α-synuclein oligomer removal system of the disclosure comprises a column having a container with an inlet and outlet for a biological fluid and a porous molded body filled into the container, a biological fluid introduction channel (circuit) through which the biological fluid is introduced into the inlet, and a biological fluid discharge channel through which the biological fluid that has passed through the column is discharged from the outlet. The biological fluid introduction channel and biological fluid discharge channel are preferably composed of flexible tubes. During use of the system, the biological fluid introduction channel may be connected in a flowable manner to the patient's body to harvest the biological fluid from the patient body. The biological fluid discharge channel may also be connected in a flowable manner to the patient's body in order to return the biological fluid to the patient's body after it has been contacted with the porous molded body.

The α-synuclein oligomer removal system can remove α-synuclein oligomers in a biological fluid by preferably 5% or greater, 10% or greater, 15% or greater, 20% or greater, 25% or greater or 30% or greater.

As mentioned above, the biological fluid is most preferably blood or plasma, in which case the system is used as a blood purification system. As an example, the blood purification system comprises a blood introduction channel comprising a flexible tube for removal of blood from a patient's body and introduction into a column, a column packed with the porous molded body of the disclosure, and a blood discharge channel comprising a flexible tube for return of blood from the column back into the body. During use of the blood purification system, the blood introduction channel and blood discharge channel are connected to a blood vessel of the patient.

The blood purification system described above is merely an example and is not limited to the described construction, and various modifications may be implemented by appropriately applying conventional techniques for blood purification. For example, the blood purification system may comprise two or more α-synuclein oligomer adsorption/removal columns, which may be connected in series or in parallel. As another example, the blood purification system may be constructed so as to comprise a plasma separator between the α-synuclein oligomer adsorption/removal column and the biological fluid introduction channel, and the plasma separated from the blood may be caused to flow through the column. More specific examples of plasma separators include membrane-type plasma separators which employ a plasma separating membrane, and centrifugal plasma separators. In this case, the blood purification system may further be constructed so that, after the α-synuclein oligomers in blood plasma have been removed, the plasma from which the α-synuclein oligomers have been removed is combined with the blood cell concentrate separated by the plasma separator, and the α-synuclein oligomer-removed blood is returned to the patient. Examples of other elements that may be incorporated into the blood purification system include elements used in conventional blood purification systems, and specifically anti-coagulation agent adding devices, pressure gauges, flow rate detectors, anomaly detectors, microparticle-removing filters, air chambers and hemolysis sensors.

### EXAMPLES

The present disclosure will now be explained in detail by Examples and Comparative Examples, with the understanding that these Examples and Comparative Examples are not intended to be limitative.

### <Evaluation and measuring methods>

### [Method of measuring removal rate of oligomers containing two or more α-synuclein monomer molecules]

A 0.18 mL portion of a porous molded body was weighed out into a 3.0 mL-volume LibraTube (product of HiPep Laboratories), and washed with phosphate buffered saline (PBS-, product of Takara Bio, Inc.) and physiological saline (Otsuka Pharmaceutical Factory, Inc.). There was then added 0.9 mL of commercially available heparinized bovine plasma or heparinized bovine whole blood, prepared to an approximately 32 ng/mL concentration of α-synuclein oligomers (Recombinant Human Alpha-synuclein protein aggregate (Active), product of Abcam Co.). After rotating and stirring for 1 hour at 37°C, 5 rpm, the α-synuclein oligomer concentration in the blood plasma of the supernatant liquid was measured by sandwich ELISA using two different antibodies (Anti-Alpha-Synuclein Filament MJFR14-6-4-2 Conformation specific, Abcam Co., Purified Mouse Antiα--Synuclein Clone 42/α-Synuclein (RUO), BD Transduction Laboratories). Defining the measured value for α-synuclein oligomers in a sample without the porous molded body as 100%, the value obtained by subtracting the residue rate (%) for α-synuclein oligomers from 100% was recorded as the α-synuclein oligomer removal rate (%) of the porous molded body. When the porous molded body fails to adsorb all or almost all α-synuclein oligomers, the removal rate may be a negative value as a result of increased sensitivity in ELISA measurement and/or the effects of variation in ELISA measurement, due to adsorption removal of plasma proteins other than α-synuclein oligomers by the porous molded body.

The molecular weights of the α-synuclein oligomers used in the test were measured using a size exclusion chromatography-multi-angle light scattering detection system (SEC-MALS), with an Optilab rEX (product of Wyatt Technology) as the differential refractometer, DAWN HELEOS (product of Wyatt Technology) as the multi-angle light scattering detector, Shodex Protein KW-804 (product of Resonac Corp.) as the separating column, and 0.3 M sodium chloride-added 0.05 M phosphate buffer (pH 7) as the eluent. As a result of the measurement, the weight-average molecular weight of the α-synuclein oligomers was 82,400 kDa.

### [Method of measuring α-synuclein monomer removal rate]

A 0.18 mL portion of a porous molded body was weighed out into a 3.0 mL-volume LibraTube (product of HiPep Laboratories), and washed with phosphate buffered saline (PBS-, product of Takara Bio, Inc.) and physiological saline (Otsuka Pharmaceutical Factory, Inc.). Next, 0.9 mL of commercially available heparinized bovine plasma or heparinized bovine whole blood prepared to an approximately 32 ng/mL concentration of α-synuclein monomers (α-Synuclein, recombinant, Human, Cosmo Bio Co., Ltd.) was added. The mixture was stirred by rotation for 1 hour at 37°C, 5 rpm, and the α-synuclein concentration in the blood plasma of the supernatant liquid was measured by sandwich ELISA using a measuring kit (Human alpha-Synuclein DuoSet ELISA, product of R&D Systems). Defining the measured value for α-synuclein monomers in a sample without the porous molded body as 100%, the value obtained by subtracting the residue rate (%) for α-synuclein monomers from 100% was recorded as the α-synuclein monomer removal rate (%) of the porous molded body. When the porous molded body fails to adsorb all or almost all α-synuclein monomers, the removal rate may be a negative value as a result of increased sensitivity in ELISA measurement and/or the effects of variation in ELISA measurement, due to adsorption removal of plasma proteins other than α-synuclein monomers by the porous molded body.

### [Method of measuring allyl group introduction into porous molded body]

The amount of allyl groups introduced (µmol/ml-wet support) was obtained by replacement of allyl groups introduced into the porous molded support with carboxyl groups and neutralization titration of the carboxyl groups. Specifically, 1 mL of the porous molded body was measured out into a 5.0 mL-volume LibraTube (HiPep Laboratories), and was mixed with 1 mL of a 25 mg/mL ammonium persulfate aqueous solution and 120 µL of thioacetic acid. The reaction product was kept at 50°C for 16 hours while stirring. After filtering the reaction mixture, the beads were washed 5 times with 10 mL of distilled water and 4 times with 10 mL of 1 M HCl water. The porous molded body was transferred to a 50 mL-volume centrifuge tube, 30 mL of 1 M HCl water was added, and the mixture was shaken and stirred for 30 minutes. The porous molded body was collected using a LibraTube and washed with 60 mL of ultrapure water (FujiFilm-Wako), after which the porous molded body was transferred to a 100 mL-volume centrifuge tube, 100 mL of ultrapure water was added, and the mixture was shaken and stirred for 30 minutes. The porous molded body was again collected using a LibraTube, and after washing with 60 mL of ultrapure water, it was transferred to a centrifuge tube, 2 mL to 500 mL of 0.01 M NaOH water was added, and the mixture was shaken and stirred for 30 minutes. After the reaction, 5 mL of supernatant was sampled, 0.1 M HCl water was added dropwise while stirring, and the amount of allyl groups introduced into the porous molded body (µmol/ml-wet support) was calculated from the drip volume at the pH inflection point. The proportion of NaOH groups ion-exchanged with the porous molded body, among the NaOH groups added to the porous molded body, was calculated as the NaOH consumption rate, with measurement of the amount of allyl group introduction judged to be suitable when the NaOH consumption rate was 20% to 40%.

### [Method of measuring amount of amine structure introduction into porous molded body]

The amount of amine structure introduced (µmol/ml-wet support) was obtained by neutralization titration of the amino groups introduced into the porous molded support. Specifically, 0.2 mL of the porous molded body was measured out into a 3.0 mL-volume LibraTube (product of HiPep Laboratories) and washed with 10 mL of physiological saline, after which the porous molded body was transferred to a 25 mL-volume centrifuge tube, 6 mL of 1 M NaOH water was added, and the mixture was shaken and stirred for 30 minutes. The porous molded body was collected using a LibraTube and washed with 12 mL of ultrapure water (FujiFilm-Wako), after which the porous molded body was transferred to a 100 mL-volume centrifuge tube, 50 mL of ultrapure water was added, and the mixture was shaken and stirred for 30 minutes. The porous molded body was again collected using a LibraTube, and after washing with 12 mL of ultrapure water, it was transferred to a centrifuge tube, 0.4 mL to 100 mL of 0.01 M HCl water was added, and the mixture was shaken and stirred for 30 minutes. After the reaction, 0.1 mL to 5 mL of supernatant was sampled, 0.1 M NaOH water was added dropwise while stirring, and the amount of amine structure introduced into the porous molded body (µmol/ml-wet support) was calculated from the drip volume at the pH inflection point. The proportion of HCl groups ion-exchanged with the porous molded body, among the HCl groups added to the porous molded body, was calculated as the HCl consumption rate, with measurement of the amount of amine structure introduction judged to be suitable when the HCl consumption rate was 20% to 40%.

### [Harmonic mean particle size of porous molded body]

The harmonic mean particle size of the porous molded body was determined by using a particle size distribution analyzer (MT3300II, product of MicrotracBEL) to measure the porous molded body after it was swelled with ultrapure water, and calculating the harmonic mean as the harmonic mean particle size (µm).

### [Specific surface area of porous molded body]

The porous molded body swelled with ultrapure water was frozen and then freeze-dried for 24 hours for drying of the porous molded body. The dried porous molded body was subjected to degassing treatment (reduced pressure drying) for 15 hours at 60°C using a VacPrep061 (product of Shimadzu-Micromeritics). A TriStarII 3020 (product of Shimadzu-Micromeritics) was then used to measure the specific surface area (m²/g) by the N₂ gas adsorption method. The value from a BET plot was used as the specific surface area. The measurement was performed 10 times, and the average value calculated for 8 points, discarding the maximum and minimum, was recorded as the specific surface area.

### <Comparative Example 1>

### [A. Introduction of allyl groups into porous molded body]

The hydroxyl groups of Sepharose 4 Fast Flow (crosslinked agarose beads, product of Cytiva) were activated with allyl bromide as a linker, in the following manner. Specifically, 15 ml of Sepharose 4 Fast Flow was suction-dried and mixed with 4.2 mL of allyl bromide, 18 mL of 6 M NaOH water and 9 ml of dimethyl sulfoxide. The mixture was stirred for 18 hours at 30°C. After filtering the mixture, the beads were successively washed with 750 ml of distilled water. Based on titration, the amount of allyl groups introduced was 108.9 (micromole)/beads (mL)-wet support.

### [B. Activation of Sepharose 4 Fast Flow by bromination]

A mixture was prepared comprising 14 ml of allyl activated Sepharose 4 Fast Flow (allyl group: 108.9 micromole allyl groups/ml-wet support), and 14 mL of 0.9 M N-bromosuccinimide (50% acetone aqueous solution). The mixture was stirred for 3 hours at 30°C. After filtering the reaction mixture, the beads were washed with 900 ml of distilled water. The activated beads were then immediately transferred into a reactor and further reacted with hexadecylamine (cetylamine) as the ligand, by the following method.

### [C. Introduction of hexadecylamine into activated beads]

An aliphatic amine structure was directly introduced into the beads via the linker structure.

Coupling of the beads was carried out by a typical procedure, using allyl group bromination and nucleophilic substitution under basic conditions. A 1 ml portion of a bromo-activated gel (allyl group introduction amount: 108.9 micromole/ml-wet support) was transferred to a reactor containing 1 mL of a 50% DMSO aqueous solution dissolving 100 mg of hexadecylamine as a ligand. The reaction product was kept at 30°C for 16 hours while stirring. After filtering the reaction mixture, the beads were washed once with 3 mL of isopropanol, once with 3 ml of distilled water, once with 3 ml of 1 M HCl water, once with 3 ml of distilled water, once with 3 mL of 1 M NaOH water and finally once with 30 mL of physiological saline, in that order. Hexadecylamine-introduced Sepharose 4 Fast Flow was obtained with an amine structure introduction amount of 6.2 micromole/ml-wet support.

Table 1 shows the adsorption performance of the obtained porous molded body for α-synuclein oligomers from blood plasma. The removal rate for α-synuclein oligomers by the porous molded body was less than 30%.

### <Example 1>

Hexadecylamine-introduced Sepharose 4 Fast Flow was obtained in the same manner as Comparative Example 1, except that 13.5 mL of an 80% isopropanol aqueous solution dissolving 3260 mg of hexadecylamine was used as the amine solution reacted with the bromo-activated gel. The amount of amine structure introduced was 39.4 micromole/ml-wet support. Table 1 shows the adsorption performance of the obtained porous molded body for α-synuclein oligomers from blood plasma. The removal rate for α-synuclein oligomers by the porous molded body was 30% or greater. It was demonstrated that the obtained porous molded body efficiently removes α-synuclein oligomers from blood plasma.

### <Comparative Example 2>

### [A. Introduction of allyl groups into porous molded body]

The hydroxyl groups of Sepharose 4 Fast Flow (crosslinked agarose beads, product of Cytiva) were activated with allyl bromide as a linker, in the following manner. Specifically, 15 ml of Sepharose 4 Fast Flow was suction-dried and mixed with 2.1 mL of allyl bromide, 9 mL of 3M NaOH water and 2.25 ml of dimethyl sulfoxide. The mixture was stirred for 18 hours at 30°C. After filtering the mixture, the beads were successively washed with 750 ml of distilled water. Based on titration, the amount of allyl groups introduced was 73.9 (micromole)/mL-wet support.

### [B. Activation of Sepharose 4 Fast Flow by bromination]

A mixture was prepared comprising 14 ml of allyl activated Sepharose 4 Fast Flow (allyl group introduction amount: 73.9 micromole/ml-wet support), and 14 mL of 0.9 M N-bromosuccinimide (50% acetone aqueous solution). The mixture was stirred for 3 hours at 30°C. After filtering the reaction mixture, the beads were washed with 900 ml of distilled water. The activated beads were then immediately transferred into a reactor and further reacted with diethylamine as the ligand, by the following method.

### [C. Introduction of diethylamine into activated beads]

Amine groups were directly introduced into the beads through the linker structure. Coupling of the beads was carried out by a typical procedure, using allyl group bromination and nucleophilic substitution under basic conditions. A 4 ml portion of a bromo-activated gel (allyl group introduction amount: 73.9 micromole/ml-wet support) was transferred to a reactor containing 4 mL of a 50% isopropanol aqueous solution dissolving 20 mg of diethylamine as a ligand. The reaction product was kept at 30°C for 16 hours while stirring. After filtering the reaction mixture, the beads were washed once with 8 mL of isopropanol, once with 4 ml of distilled water, once with 4 ml of 1 M HCl water, once with 4 ml of distilled water, once with 4 mL of 1 M NaOH water and finally once with 120 mL of physiological saline, in that order. Diethylamine-introduced Sepharose 4 Fast Flow was obtained with an amine structure introduction amount of 5.5 micromole/ml-wet support.

Table 1 shows the adsorption performance of the obtained porous molded body for α-synuclein oligomers from blood plasma. The removal rate for α-synuclein oligomers in the porous molded body was less than 30%.

### <Comparative Example 3>

Diethylamine-introduced Sepharose 4 Fast Flow was obtained in the same manner as Comparative Example 2, except that 40 mg of diethylamine was used as the amine reacted with the bromo-activated gel. The amount of amine structure introduced was 16.7 micromole/ml-wet support. Table 1 shows the adsorption performance of the obtained porous molded body for α-synuclein oligomers from blood plasma. The removal rate for α-synuclein oligomers in the porous molded body was less than 30%.

### <Comparative Example 4>

Diethylamine-introduced Sepharose 4 Fast Flow was obtained in the same manner as Comparative Example 2, except that 80 mg of diethylamine was used as the amine reacted with the bromo-activated gel. The amount of amine structure introduced was 28.9 micromole/ml-wet support. Table 1 shows the adsorption performance of the obtained porous molded body for α-synuclein oligomers from blood plasma. The removal rate for α-synuclein oligomers in the porous molded body was less than 30%.

### <Example 2>

Diethylamine-introduced Sepharose 4 Fast Flow was obtained in the same manner as Comparative Example 2, except that 100 mg of diethylamine was used as the amine reacted with the bromo-activated gel. The amount of amine structure introduced was 51.0 micromole/ml-wet support. Table 1 shows the adsorption performance of the obtained porous molded body for α-synuclein oligomers from blood plasma. The removal rate for α-synuclein oligomers by the porous molded body was 30% or greater. It was demonstrated that the obtained porous molded body efficiently removes α-synuclein oligomers from blood plasma.

### <Example 3>

Diethylamine-introduced Sepharose 4 Fast Flow was obtained in the same manner as Comparative Example 1, except that 1 mL of a 50% isopropanol aqueous solution dissolving 500 mg of diethylamine was used as the amine solution reacted with the bromo-activated gel. The amount of amine structure introduced was 95.3 micromole/ml-wet support. Table 1 shows the adsorption performance of the obtained porous molded body for α-synuclein oligomers from blood plasma. The removal rate for α-synuclein oligomers by the porous molded body was 30% or greater. It was demonstrated that the obtained porous molded body efficiently removes α-synuclein oligomers from blood plasma.

### <Example 4>

Ethylamine-introduced Sepharose 4 Fast Flow was obtained in the same manner as Comparative Example 1, except that 1 mL of a 50% isopropanol aqueous solution dissolving 77.0 mg of ethylamine was used as the amine solution reacted with the bromo-activated gel. The amount of amine structure introduced was 53.2 micromole/ml-wet support. Table 1 shows the adsorption performance of the obtained porous molded body for α-synuclein oligomers from blood plasma. The removal rate for α-synuclein oligomers by the porous molded body was 30% or greater. It was demonstrated that the obtained porous molded body efficiently removes α-synuclein oligomers from blood plasma.

### <Example 5>

Triethylamine-introduced Sepharose 4 Fast Flow was obtained in the same manner as Comparative Example 1, except that 1 mL of a 50% isopropanol aqueous solution dissolving 691.4 mg of triethylamine was used as the amine solution reacted with the bromo-activated gel.

The amount of amine structure introduced was 53.7 micromole/ml-wet support. Table 1 shows the adsorption performance of the obtained porous molded body for α-synuclein oligomers from blood plasma. The removal rate for α-synuclein oligomers by the porous molded body was 30% or greater. It was demonstrated that the obtained porous molded body efficiently removes α-synuclein oligomers from blood plasma.

### <Example 6>

N-benzyl-N-methyl-ethanolamine-introduced Sepharose 4 Fast Flow was obtained in the same manner as Comparative Example 1, except that 1 mL of a 50% isopropanol aqueous solution dissolving 2259.5 mg of N-benzyl-N-methyl-ethanolamine was used as the amine solution reacted with the bromo-activated gel. The amount of amine structure introduced was 49.2 micromole/ml-wet support. Table 1 shows the adsorption performance of the obtained porous molded body for α-synuclein oligomers from blood plasma. The removal rate for α-synuclein oligomers by the porous molded body was 30% or greater. It was demonstrated that the obtained porous molded body efficiently removes α-synuclein oligomers from blood plasma.

### <Example 7>

N,N-dimethyl-2-phenoxyethylamine-introduced Sepharose 4 Fast Flow was obtained in the same manner as Example 1, except that 1 mL of a 50% isopropanol aqueous solution dissolving 141.2 mg of N,N-dimethyl-2-phenoxyethylamine was used as the amine solution reacted with the bromo-activated gel. The amount of amine structure introduced was 59.4 micromole/ml-wet support. Table 2 shows the adsorption performance of the obtained porous molded body for α-synuclein oligomers from blood plasma. The removal rate for α-synuclein oligomers by the porous molded body was 30% or greater. It was demonstrated that the obtained porous molded body efficiently removes α-synuclein oligomers from blood plasma.

### <Example 8>

Butylamine-introduced Sepharose 4 Fast Flow was obtained in the same manner as Comparative Example 1, except that 1 mL of a 50% isopropanol aqueous solution dissolving 987.4 mg of butylamine was used as the amine solution reacted with the bromo-activated gel. The amount of amine structure introduced was 68.3 micromole/ml-wet support. Table 2 shows the adsorption performance of the obtained porous molded body for α-synuclein oligomers from blood plasma. The removal rate for α-synuclein oligomers by the porous molded body was 30% or greater. It was demonstrated that the obtained porous molded body efficiently removes α-synuclein oligomers from blood plasma.

### <Example 9>

N,N-dimethylbutylamine-introduced Sepharose 4 Fast Flow was obtained in the same manner as Example 1, except that 1 mL of an 80% isopropanol aqueous solution dissolving 379.4 mg of N,N-dimethylbutylamine was used as the amine solution reacted with the bromo-activated gel. The amount of amine structure introduced was 51.0 micromole/ml-wet support. Table 2 shows the adsorption performance of the obtained porous molded body for α-synuclein oligomers from blood plasma. The removal rate for α-synuclein oligomers by the porous molded body was 30% or greater. It was demonstrated that the obtained porous molded body efficiently removes α-synuclein oligomers from blood plasma.

### <Example 10>

N,N-dimethylhexylamine-introduced Sepharose 4 Fast Flow was obtained in the same manner as Example 1, except that 1 mL of an 80% isopropanol aqueous solution dissolving 161.6 mg of N,N-dimethylhexylamine was used as the amine solution reacted with the bromo-activated gel. The amount of amine structure introduced was 40.4 micromole/ml-wet support. Table 2 shows the adsorption performance of the obtained porous molded body for α-synuclein oligomers from blood plasma. The removal rate for α-synuclein oligomers by the porous molded body was 30% or greater. It was demonstrated that the obtained porous molded body efficiently removes α-synuclein oligomers from blood plasma.

### <Example 11>

### [A. Introduction of allyl groups into porous molded body]

The hydroxyl groups of Cellfine GH-25 (crosslinked cellulose beads, product of JNC Corp.) were activated with allyl bromide as a linker, in the following manner. Specifically, 18 ml of Sepharose 4 Fast Flow was suction-dried and mixed with 5.0 mL of allyl bromide, 21.6 mL of 6 M NaOH water and 10.8 ml of dimethyl sulfoxide. The mixture was stirred for 18 hours at 30°C. After filtering the mixture, the beads were successively washed with 900 ml of distilled water. Based on titration, the amount of allyl groups introduced was 132.6 (micromole)/mL-wet support.

### [B. Activation of Cellfine GH-25 by bromination]

A mixture was prepared comprising 17 ml of allyl activated Cellfine GH-25 (allyl group introduction amount: 132.6 micromole/ml-wet support), and 17 mL of 0.9 M N-bromosuccinimide (50% acetone aqueous solution). The mixture was stirred for 3 hours at 30°C. After filtering the reaction mixture, the beads were washed with 800 ml of distilled water. The activated beads were then immediately transferred into a reactor and further reacted with diethylamine as the ligand, by the following method.

### [C. Introduction of diethylamine into activated beads]

Amine groups were directly introduced into the beads through the linker structure. Coupling of the beads was carried out by a typical procedure, using allyl group bromination and nucleophilic substitution under basic conditions. A 1 ml portion of a bromo-activated gel (allyl group introduction amount: 132.6 micromole/ml-wet support) was transferred to a reactor containing 1 mL of a 50% isopropanol aqueous solution dissolving 15.6 mg of diethylamine as a ligand. The reaction product was kept at 25°C for 16 hours while stirring. After filtering the reaction mixture, the beads were washed once with 3 mL of isopropanol, once with 3 ml of distilled water, once with 3 ml of 1 M HCl water, once with 3 ml of distilled water, once with 3 mL of 1 M NaOH water and finally once with 30 mL of physiological saline, in that order. Diethylamine-introduced Sepharose 4 Fast Flow gel was obtained with an amine structure introduction amount of 67.4 micromole/ml-wet support.

Table 2 shows the adsorption performance of the obtained porous molded body for α-synuclein oligomers from blood plasma. The removal rate for α-synuclein oligomers by the porous molded body was 30% or greater. It was demonstrated that the obtained porous molded body efficiently removes α-synuclein oligomers from blood plasma.

### <Example 12>

Diethylamine-introduced Sepharose 4 Fast Flow was obtained in the same manner as Example 11, except that Cellfine GCL-2000F (crosslinked cellulose beads, product of JNC Corp.) was used as the porous molded body and 1 mL of a 50% isopropanol aqueous solution dissolving 20.8 mg of diethylamine was used as the amine solution reacted with the bromo-activated gel. The amount of amine structure introduced was 50.6 micromole/ml-wet support. Table 2 shows the adsorption performance of the obtained porous molded body for α-synuclein oligomers from blood plasma. The removal rate for α-synuclein oligomers by the porous molded body was 30% or greater. It was demonstrated that the obtained porous molded body efficiently removes α-synuclein oligomers from blood plasma.

### <Example 13>

Plasorba BR (polystyrene-based beads by Asahi Kasei Medical Co., Ltd.) was used as the porous molded body. The porous molded body has a methylene group as the linker structure and a trimethylamine group as the aliphatic amine structure. The amount of amine structure introduced was 346.8 micromole/ml-wet support. The harmonic mean particle size of the porous molded body was 396 µm, and the specific surface area was 24.0 m²/g. The removal rate for α-synuclein oligomers from blood plasma by the porous molded body was 30% or greater. It was demonstrated that the obtained porous molded body efficiently removes α-synuclein oligomers from blood plasma. The removal rate for α-synuclein monomers from blood plasma by the porous molded body was also 30% or greater. It was demonstrated that the obtained porous molded body efficiently removes α-synuclein monomers from blood plasma. The porous molded body also had an excellent balance between adsorption efficiency and mechanical stability, and an excellent balance between flow property and adsorption efficiency.

### <Example 14>

The porous molded body used was DIAION WA20 (polystyrene-based beads by Mitsubishi Chemical Holdings Corp.). The porous molded body has methylene as the linker structure and diethylenetriamine (-NH(CH₂CH₂NH)₂H) as the aliphatic amine structure. The amount of amine structure introduced was 733.6 micromole/ml-wet support. The harmonic mean particle size of the porous molded body was 569 µm, and the specific surface area was 2.3 m²/g. The removal rate for α-synuclein oligomers from blood plasma by the porous molded body was 30% or greater. It was demonstrated that the obtained porous molded body efficiently removes α-synuclein oligomers from blood plasma. The porous molded body also had an excellent balance between adsorption efficiency and mechanical stability, and an excellent balance between flow property and adsorption efficiency.

### <Example 15>

The porous molded body used was DIAION WA30 (polystyrene-based beads by Mitsubishi Chemical Holdings Corp.). The porous molded body has a methylene group as the linker structure and dimethylamine as the aliphatic amine structure. The amount of amine structure introduced was 932.3 micromole/ml-wet support. The harmonic mean particle size of the porous molded body was 582 µm. The removal rate for α-synuclein oligomers from blood plasma by the porous molded body was 30% or greater. It was demonstrated that the obtained porous molded body efficiently removes α-synuclein oligomers from blood plasma. The porous molded body had an excellent balance between flow property and adsorption efficiency.

### <Example 16>

Plasorba BR (polystyrene-based beads by Asahi Kasei Medical Co., Ltd.) was used as the porous molded body. The porous molded body has a methylene group as the linker and a trimethylamine group as the aliphatic amine structure. The amount of amine structure introduced was 346.8 micromole/ml-wet support. The harmonic mean particle size of the porous molded body was 396 µm, and the specific surface area was 24.0 m²/g. The removal rate for α-synuclein oligomers from blood by the porous molded body was 30% or greater. It was demonstrated that the obtained porous molded body efficiently removes α-synuclein oligomers from blood. The porous molded body also had an excellent balance between adsorption efficiency and mechanical stability, and an excellent balance between flow property and adsorption efficiency.

### [Table 1]

**Table 1**

| | Comp. Example 1 | Example 1 | Comp. Example 2 | Comp. Example 3 | Comp. Example 4 | Example 2 | Example 3 | Example 4 | Example 5 | Example 6 |
|---|---|---|---|---|---|---|---|---|---|---|
| Porous molded body material | Crosslinked agarose | Crosslinked agarose | Crosslinked agarose | Crosslinked agarose | Crosslinked agarose | Crosslinked agarose | Crosslinked agarose | Crosslinked agarose | Crosslinked agarose | Crosslinked agarose |
| Linker structure | 2-Hydroxypropylene | 2-Hydroxypropylene | 2-Hydroxypropylene | 2-Hydroxypropylene | 2-Hydroxypropylene | 2-Hydroxypropylene | 2-Hydroxypropylene | 2-Hydroxypropylene | 2-Hydroxypropylene | 2-Hydroxypropylene |
| Aliphatic amine structure (excluding linker structure) | Hexadecylamine | Hexadecylamine | Diethylamine | Diethylamine | Diethylamine | Diethylamine | Diethylamine | Ethylamine | Triethylamine | N-Benzyl-N-methyl-ethanolamine |
| Amount of amine structure introduced (µmol/ml-wet support) | 6.2 | 39.4 | 5.5 | 16.7 | 28.9 | 51.0 | 95.3 | 53.2 | 53.7 | 49.2 |
| Mean particle size (µm) | Unmeasured | Unmeasured | Unmeasured | Unmeasured | Unmeasured | Unmeasured | Unmeasured | Unmeasured | Unmeasured | Unmeasured |
| Specific surface area (m²/g) | Unmeasured | Unmeasured | Unmeasured | Unmeasured | Unmeasured | Unmeasured | Unmeasured | Unmeasured | Unmeasured | Unmeasured |
| Biological fluid | Blood plasma | Blood plasma | Blood plasma | Blood plasma | Blood plasma | Blood plasma | Blood plasma | Blood plasma | Blood plasma | Blood plasma |
| α-Synuclein oligomer removal rate (%) | -8.9 | 53.4 | -5.6 | 18.2 | 7.3 | 72.5 | 65.8 | 59.9 | 66.3 | 37.2 |
| α-Synuclein monomer removal rate (%) | Unmeasured | Unmeasured | Unmeasured | Unmeasured | Unmeasured | Unmeasured | Unmeasured | Unmeasured | Unmeasured | Unmeasured |

### [Table 2]

**Table 2**

| | Example 7 | Example 8 | Example 9 | Example 10 | Example 11 | Example 12 | Example 13 | Example 14 | Example 15 | Example 16 |
|---|---|---|---|---|---|---|---|---|---|---|
| Porous molded body material | Crosslinked agarose | Crosslinked agarose | Crosslinked agarose | Crosslinked agarose | Cellulose 25-GH | Cellulose 2000F | Styrene-based polymer | Styrene-based polymer | Styrene-based polymer | Styrene-based polymer |
| Linker structure | 2-Hydroxypropylene | 2-Hydroxypropylene | 2-Hydroxypropylene | 2-Hydroxypropylene | 2-Hydroxypropylene | 2-Hydroxypropylene | Methylene | Methylene | Methylene | Methylene |
| Aliphatic amine structure (excluding linker structure) | N,N-Dimethyl-2-phenoxy-ethylamine | Butylamine | N,N-Dimethylbutylamine | N,N-Dimethyl-hexylamine | Diethylamine | Diethylamine | Trimethylamine | Diethylenetriamine | Dimethylamine | Trimethylamine |
| Amount of amine structure introduced (µmol/ml-wet support) | 59.4 | 68.3 | 51.0 | 40.4 | 67.4 | 50.6 | 346.8 | 733.6 | 932.3 | 346.8 |
| Harmonic mean particle size (µm) | Unmeasured | Unmeasured | Unmeasured | Unmeasured | Unmeasured | Unmeasured | 396 | 569 | 582 | 396 |
| Specific surface area (m²/g) | Unmeasured | Unmeasured | Unmeasured | Unmeasured | Unmeasured | Unmeasured | 24.0 | 2.3 | Unmeasured | 24.0 |
| Biological fluid | Blood plasma | Blood plasma | Blood plasma | Blood plasma | Blood plasma | Blood plasma | Blood plasma | Blood plasma | Blood plasma | Blood |
| α-Synuclein oligomer removal rate (%) | 32.7 | 66.9 | 69.5 | 65.9 | 75.9 | 53.8 | 70.4 | 96.7 | 66.5 | 80.7 |
| α-Synuclein monomer removal rate (%) | Unmeasured | Unmeasured | Unmeasured | Unmeasured | Unmeasured | Unmeasured | 92.3 | Unmeasured | Unmeasured | Unmeasured |

### INDUSTRIAL APPLICABILITY

The porous molded body of the disclosure can be suitably used for efficient removal of α-synuclein oligomers from biological fluids.

## Claims

1. A porous molded body for removal of α-synuclein oligomers, comprising a porous molded support having a positive charge derived from an amine structure, wherein the porous molded support comprises the amine structure at 30 µmol/ml-wet support or greater and 10,000 µmol/ml-wet support or less.

2. The porous molded body according to claim 1, wherein the amine structure is an aliphatic amine structure.

3. The porous molded body according to claim 1 or 2, wherein the amine structure is a secondary, tertiary or quaternary amine, or a combination thereof, and the amine group has 1 to 6 carbon atoms.

4. The porous molded body according to claim 1 or 2, wherein the amine structure includes a quaternary amine.

5. The porous molded body according to claim 1 or 2, wherein the porous molded support is made of crosslinked agarose, crosslinked cellulose or a polystyrene-based polymer, or a combination thereof.

6. The porous molded body according to claim 1 or 2, wherein the harmonic mean particle size of the porous molded support is 100 µm to 800 µm.

7. The porous molded body according to claim 1 or 2, wherein the specific surface area of the porous molded support is 1 m²/g to 100 m²/g.

8. An α-synuclein oligomer removal system, the system comprising:
a column having a container with an inlet and outlet for a biological fluid, and a porous molded body filled into the container,
a biological fluid introduction channel through which the biological fluid is introduced into the inlet, and
a biological fluid discharge channel through which the biological fluid that has passed through the column is discharged from the outlet,
wherein the porous molded body comprises a porous molded support having a positive charge derived from an amine structure, the porous molded support having the amine structure at 30 µmol/ml-wet support or greater and 10,000 µmol/ml-wet support or less.

9. The system according to claim 8, wherein the biological fluid is blood, and a plasma separator is provided between the column and the biological fluid introduction channel, the construction being such that plasma separated from the blood flows through the column.
